(19) Europäisches Patentamt
European Patent Office
Office européen des brevets

(11) Veröffentlichungsnummer: **0 056 446**
**A2**

(12) # EUROPÄISCHE PATENTANMELDUNG

(21) Anmeldenummer: 81109065.3

(22) Anmeldetag: 28.10.81

(51) Int. Cl.³: **C 07 C 157/12**
**A 01 N 47/44**

(30) Priorität: 15.01.81 DE 3101121

(43) Veröffentlichungstag der Anmeldung:
28.07.82 Patentblatt 82/30

(84) Benannte Vertragsstaaten:
AT BE CH DE FR GB IT LI NL

(71) Anmelder: **WACKER-CHEMIE GMBH**
**Prinzregentenstrasse 22**
**D-8000 München 22(DE)**

(72) Erfinder: **Kinzel, Peter, Ing.-grad.**
**Jägerkampstrasse 21a**
**D-8152 Westerham-Feldkirchen(DE)**

(72) Erfinder: **Mack, Wilhelm, Dr. Dipl.-Chem.**
**Unghausen 24**
**D-8263 Burghausen(DE)**

(72) Erfinder: **Müller, Frank, Dr. Dipl.-Chem.**
**Korbinian-Westermair-Strasse 9**
**D-8011 Siegertsbrunn(DE)**

(54) **Aromatisch substituierte Formamidino-Thioharnstoffe als herbizide Mittel.**

(57) Die Erfindung betrifft herbizide Mittel und deren Herstellung aus der Reihe aromatisch substituierter Formamidino-Thioharnstoffe der allgemeinen Formel

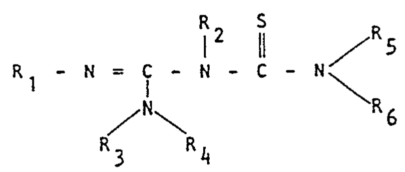

wobei

$R_1$ und $R_2$ gleiche oder verschiedene, ggf. substituierte aromatische Reste darstellen und
$R_3$, $R_4$, $R_5$ und $R_6$ für gleiche oder verschiedene aliphatische Reste mit bis zu 4 Kohlenstoffatomen, cycloaliphatische Reste mit 5 bis 6 Kohlenstoffatomen stehen und weiterhin $R_3$ und $R_4$ sowie $R_5$ und $R_6$, ggf. ein weiteres Heteroatom enthaltende Ringe mit 4 bis 5 Kohlenstoffatomen bilden können.

Die erfindungsgemäßen Wirkstoffe können zur selektiven Bekämpfung mono- und dikotyler Schadpflanzen in Kulturpflanzungen eingesetzt werden.

EP 0 056 446 A2

Croydon Printing Company Ltd.

0056446

WACKER-CHEMIE
GMBH

München, den 15.12.1980
LC-PAT/Dr.Ra/we

Wa 8050-C

## Aromatisch substituierte Formamidino-Thioharnstoffe als herbizide Mittel

Durch die Veröffentlichung von P.K. Srivastava im Indian J. Chem. 1963, Seiten 354 bis 358 sind Verbindungen aus der Gruppe aromatisch substituierter Formamidino-Thioharnstoffe bekanntgeworden. Beispielsweise wird der 1-Phenyl-1-(N-Phenyl-N,N'-dimethylformamidino)-3,3-dimethyl-thioharnstoff erwähnt. Die genannte Verbindung wird durch Oxidation von 1-Phenyl-3,3-dimethyl-thioharnstoff mit Brom erhalten.

Einen anderen Syntheseweg für die obengenannten Formamidino-Thioharnstoffe geben Paul Held et al in Z. Chem. 13, 1973, Seite 341 bis 342 an, wobei $N^2$-Phenyl-$N^1$,$N^1$-dimethyl-chlor-formamidin mit 1-Phenyl-3,3-dimethyl-thioharnstoff zum Zielprodukt umgesetzt wird.

Aufgabe der Erfindung war es, herbizide Mittel zur selektiven Bekämpfung unerwünschter Pflanzen in Kulturpflanzungen vorzustellen.

Es wurde nun gefunden, daß eine Auswahl von aromatisch substituierten Formamidino-Thioharnstoffen herbizide Eigenschaften besitzen. Überraschenderweise zeigen diese Mittel bei tolerantem Verhalten gegenüber einer Vielzahl von Kulturpflanzen hohe herbizide Wirksamkeit gegenüber in derartigen Kulturpflanzungen häufig vorkommenden mono- und dikotylen Schadpflanzen.

Gegenstand der Erfindung sind herbizid wirksame Zusammensetzungen, die zumindest eine Verbindung der allgemeinen Formel

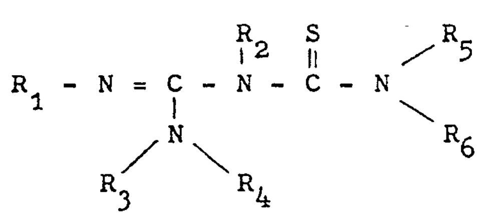

enthalten, wobei

$R_1$ und $R_2$ gleiche oder verschiedene, ggf. substituierte aromatische Reste sind, und

$R_3$, $R_4$, $R_5$ und $R_6$ für gleiche oder verschiedene Substituenten stehen, mit der Bedeutung aliphatischer Rest mit 1 bis 4 Kohlenstoffatomen, cycloaliphatischer Rest mit 5 bis 6 Kohlenstoffatomen und weiterhin, daß $R_3$ und $R_4$ sowie $R_5$ und $R_6$, ggf. ein weiteres Heteroatom enthaltende Ringe mit 4 bis 5 Kohlenstoffatomen bilden können.

Vorzugsweise bedeuten $R_1$ und $R_2$ gleich oder verschiedene, ggf. substituierte Phenyl- und Naphtylreste, insbesondere ggf. substituierte Phenylreste.

Als Substitutionstyp für die obengenannten aromatischen Reste kommen bevorzugt 0 bis 3, insbesondere 0 bis 2, weitere Substituenten in Betracht aus der Gruppe der Halogene, insbesondere des Chlors und des Fluors, der Alkylgruppen mit 1 bis 3 Kohlenstoffatomen, der substituierten Alkylgruppen mit 1 bis 3 Kohlenstoffatomen, der Alkoxygruppen mit 1 bis 3 Kohlenstoffatomen, der Mercaptogruppen mit 1 bis 3 Kohlenstoffatomen, der Carboxyalkylgruppen mit 1 bis 3 Kohlenstoffatomen, der Nitro- und der Nitrilgruppen.

Beispiele für die obengenannten substituierten Alkylgruppen mit 1 bis 3 Kohlenstoffatomen sind Trifluormethyl, 3.3.3-trifluorpropyl, Chlor-difluor-methyl, Chlormethyl, 1.2-Dichlorethyl, Hydroxymethyl, Mercaptomethyl u.a.

Sofern $R_3/R_4$ bzw. $R_5/R_6$ einen, ggf. ein weiteres Heteroatom enthaltenden Ring bilden mit 4 bis 5 Kohlenstoffatom, sind tertiär gebundener Stickstoff, Schwefel und Sauerstoff als Heteroatom bevorzugt. Beispiele für diesen Ringsubstitutionstyp sind der Piperidin-, der N-Methyl-Piperazin-, der Morpholin- und der Thiazinrest.

Bevorzugt sind $R_3$, $R_4$, $R_5$ und $R_6$ Methylreste.

Weiterhin sind aufgrund der leichteren chemischen Zugänglichkeit solche erfindungsgemäßen Wirkstoffe bevorzugt, bei denen $R_1$ und $R_2$ sowie die Kombinationen $R_3/R_4$ und $R_5/R_6$ identisch sind. Derartige Verbindungen sind in an sich bekannter Weise durch Kondensation entsprechend substituierter Thioharnstoffe in Gegenwart von Brom, Chlor oder Schwefelhalogeniden, wie $SOCl_2$, $SO_2Cl_2$, $S_2Cl_2$ u.a. zugänglich.

Ausgangssubstanzen für den obengenannten Reaktionsweg bilden demnach Thioharnstoffe der allgemeinen Formel

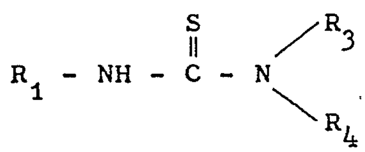

bzw.

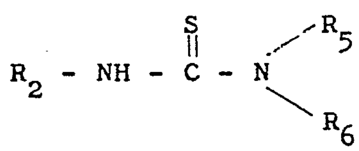

Ein bevorzugtes Herstellungsverfahren für erfindungsgemäße Wirkstoffe der allgemeinen Formel

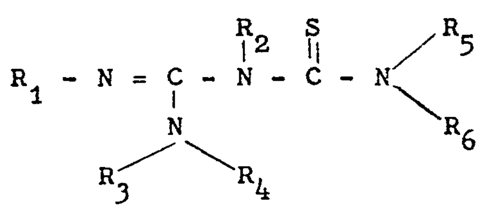

wobei

$R_1$ und $R_2$ gleiche, ggf. substituierte aromatische Reste bedeuten und

$R_3$, $R_4$, $R_5$ und $R_6$, mit der Maßgabe, daß die Kombinationen $R_3/R_4$ und $R_5/R_6$ identisch sind, für aliphatische Reste mit 1 bis 4 Kohlenstoffatomen, für cycloaliphatische Reste mit 5 bis 6 Kohlenstoffatomen

stehen, sowie $R_3/R_4$ bzw. $R_5/R_6$ einen, ggf. ein weiteres Heteroatom enthaltenden Ring mit 4 bis 5 Kohlenstoffatomen bilden können,

ist dadurch gekennzeichnet, daß

a) Thioharnstoff der allgemeinen Formel

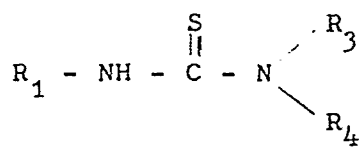

mit Chlor oder Brom in Gegenwart polarer Lösungsmittel als Flüssigphase behandelt wird und

b) das Reaktionsprodukt gemäß a) alkalisch gestellt wird.

Die Reaktionstemperaturen betragen 0 bis 60 °C, bevorzugt 15 bis 25 °C.

Die Zugabe von Brom oder Chlor erfolgt am besten in annähernd stöchiometrischen Mengen, d.h. 2 Mol Thioharnstoff werden mit annähernd 1 Mol Chlor oder Brom umgesetzt.

Als polare Lösungsmittel eignen sich Methanol, Ethanol, Propanol, Isopropanol sowie deren Gemische.

Die Kondensationsreaktion erfolgt unter Abscheidung von elementarem Schwefel. Zur besseren Filtrierbarkeit des Schwefels wird das Reaktionsgemisch nach Beendigung der Reaktion zweckmäßigerweise kurz aufgekocht und anschließend filtriert. Schließlich wird die Lösung alkalisch gestellt, wobei das Zielprodukt als Niederschlag erhalten wird. Als Basen dienen NaOH, KOH, Soda und dergleichen.

Ein allgemein anwendbares Herstellungsverfahren, nach dem insbesondere auch solche erfindungsgemäßen Wirkstoffe zugänglich sind mit jeweils nicht identischen Substituenten $R_1$ und $R_2$ sowie nicht identischen Kombinationen $R_3/R_4$ und $R_5/R_6$ eröffent sich durch die an sich bekannte Umsetzung entsprechend substituierten Harnstoffs mit entsprechend substituiertem Chlor-Formamidin.

Das genannte Verfahren zur Herstellung von Verbindungen der allgemeinen Formel

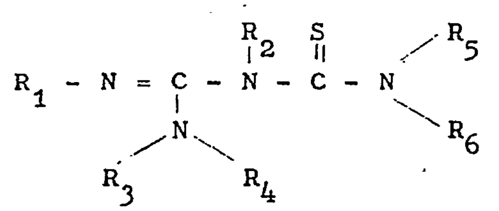

wobei

$R_1$ und $R_2$ gleiche oder verschiedene, ggf. substituierte aromatische Reste sind und

$R_3$, $R_4$, $R_5$ und $R_6$ für gleiche oder verschiedene Substituenten stehen mit der Bedeutung aliphatischer Rest mit 1 bis 4 Kohlenstoffatomen, cycloaliphatischer Rest mit 5 bis 6 Kohlenstoffatomen und weiterhin, daß $R_3$ und $R_4$ sowie $R_5$ und $R_6$, ggf. ein weiteres Heteroatom enthaltende Ringe mit 4 bis 5 Kohlenstoffatomen bilden können,

ist dadurch gekennzeichnet, daß

a) Chlorformamidin der allgemeinen Formel

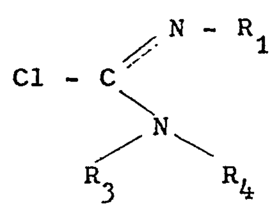

wobei $R_1$, $R_3$ und $R_4$ die obengenannte Bedeutung haben

mit Thioharnstoff der allgemeinen Formel

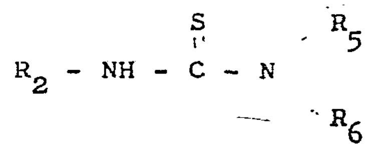

wobei

$R_2$, $R_5$ und $R_6$ die obengenannte Bedeutung haben

in Gegenwart von Chloroform umgesetzt werden und

b) das Reaktionsgemisch gemäß a) alkalisch gestellt wird.

Typischerweise wird dabei so vorgegangen, daß ein in etwa stöchiometrischer Ansatz von Chlorformamidin und Thioharnstoff in Chloroform gelöst bei Temperaturen von 15 bis 30 °C 12 bis 30 Stunden stehengelassen wird. Danach wird das Chloroform abgezogen, der Rückstand in wenig Alkohol, beispielsweise in Methanol, gelöst und anschließend die Lösung durch Zugabe von Lauge alkalisch gestellt. Schließlich wird die alkalische Lösung in Wasser eingetragen, wobei das Zielprodukt als Niederschlag anfällt.

Die als Ausgangssubstanzen einzusetzenden Chlorformamidine sind in an sich bekannter Weise durch Umsetzen von Harnstoff mit Phosphorpentachlorid zugänglich. Hierzu sei beispielsweise auf die Vorschrift J. Burmann, Bl. Soc. Neuchatel Sci. Nat. 37, Seite 188 (1909) verwiesen.

Die als Ausgangssubstanzen einzusetzenden Thioharnstoffe sind beispielsweise durch Umsetzen entsprechend substituierter aromatischer Isothiocyanate mit sekundärem Amin zugänglich. Die aromatischen Isothiocyanate sind wiederum aus aromatischem primärem Amin und Schwefelkohlenstoff darstellbar.

Beispiele für erfindungsgemäße herbizide Wirkstoffe sind:

N- [(dimethylamino)(phenylimino)methyl]-N',N'-dimethyl-N-phenyl-thioharnstoff

N- [(diethylamino)(phenylimino)methyl]-N',N'-dimethyl-N-phenyl-thioharnstoff

N- [(dipropylamino)(phenylimino)methyl]-N',N'-dimethyl-N-phenyl-thioharnstoff

N- [(dibutylamino)(phenylimino)methyl]-N',N'-dimethyl-N-phenyl-thioharnstoff

N- [(ethylmethylamino)(phenylimino)methyl]-N',N'-dimethyl-N-phenyl-thioharnstoff

N- [(diethylamino)(phenylimino)methyl]-N',N'-diethyl-N-phenyl-thioharnstoff

N- [(dipropylamino)(phenylimino)methyl]-N',N'-dipropyl-N-phenyl-thoharnstoff

N- [(dimethylamino)(phenylimino)methyl]-N',N'-dibutyl-N-phenyl-thioharnstoff

N- [(dimethylamino)(p-chlorphenylimino)methyl]-N',N'-dimethyl-N-phenyl-thioharnstoff

N- [(dimethylamino)(p-chlorphenylimino)methyl]-N',N'-dimethyl-N-p-chlorphenyl-thioharnstoff

N- [(dimethylamino)(3.4-dichlorphenylimino)methyl]-N',N'-dimethyl-N-phenyl-thioharnstoff

N- [(dimethylamino)(3.4-dichlorphenylimino)methyl]-N',N'-dimethyl-N-3.4-dichlorphenyl)-thioharnstoff

N- [(dimethylamino)(p-fluorphenylimino)methyl]-N',N'-dimethyl-N-phenyl-thioharnstoff

N- [(dimethylamino)(p-fluorphenylimino)methyl]-N',N'-dimethyl-N-p-fluorphenyl-thioharnstoff

N- [(dimethylamino)(3-chlor-4-fluorphenylimino)methyl]-N',N'-dimethyl-N-(3-chlor-4-fluorphenyl)-thioharnstoff

N- [(dimethylamino)(3-chlor-4-fluorphenylimino)methyl]-N',N'-dimethyl-N-phenyl-thioharnstoff

N- [(dimethylamino)(p-tolylimino)methyl]-N',N'-dimethyl-N-phenyl-thioharnstoff

N- [(dimethylamino)(p-tolylimino)methyl]-N',N'-dimethyl-N-p-tolyl-thioharnstoff

N- [(dimethylamino)(2.4-dimethyl-phenylimino)methyl]-N',N'-dimethyl-N-phenyl-thioharnstoff

N- [(dimethylamino)(2.4-dimethyl-phenylimino)methyl]-N',N'-dimethyl-N-2.4-dimethyl-phenyl-thioharnstoff

N- [(dimethylamino)(2-methyl-4-chlorphenylimino)methyl]-N',N'-dimethyl-N-phenyl-thioharnstoff

N- [(dimethylamino)(2-methyl-4-chlorphenylimino)methyl]-N',N'-dimethyl-N-(2-methyl-4-chlorphenyl)-thioharnstoff

N- [(dimethylamino)(2.4.6-trimethyl-phenylimino)methyl]-N',N'-dimethyl-N-phenyl-thioharnstoff

N- [(diethylamino)(4-ethyl-phenylimino)methyl]-N',N'-dimethyl-N-phenyl-thioharnstoff

N- [(dimethylamino)(4-i-propyl-phenylimino)methyl]-N',N'-dimethyl-N-phenyl-thioharnstoff

N- [(dimethylamino)(4-i-propyl-phenylimino)methyl]-N',N'-dimethyl-N-4-i-propyl-phenyl)-thioharnstoff

N- [(dimethylamino)(4-methoxymethyl-phenylimino)methyl]-N',N'-dimethyl-N-(4-i-propyl-phenyl)-thioharnstoff

N- [(dimethylamino)(4-methoxymethyl-phenylimino)methyl]-N',N'-dimethyl-N-(4-hydroxymethyl-phenyl)-thioharnstoff

N- [(dimethylamino)(4-trifluormethyl-phenylimino)methyl]-N',N'-dimethyl-N-(4-hydroxymethyl-phenyl)-thioharnstoff

N- [(dimethylamino)(4-trifluormethyl-phenylimino)methyl]-N',N'-dimethyl-N-(4-trifluormethyl-phenyl)-thioharnstoff

N- [(dimethylamino)[4-(3.3.3-trifluorpropyl-phenylimino)]methyl]-N',N'-dimethyl-N-phenyl-thioharnstoff

N- [(dimethylamino)(4-nitro-phenylimino)methyl]-N',N'-dimethyl-N-phenyl-thioharnstoff

N- [(dimethylamino)(4-nitro-phenylimino)methyl]-N',N'-dimethyl-N-(4-nitrophenyl)-thioharnstoff

N- [(dimethylamino)(2.4-dinitro-phenylimino)methyl]-N',N'-dimethyl-N-phenyl-thioharnstoff

N- [(dimethylamino)(2.4-dinitro-phenylimino)methyl]-N',N'-dimethyl-N-(2.4-dinitrophenyl)-thioharnstoff

N- [(dimethylamino)(4-nitrilo-phenylimino)methyl]-N',N'-dimethyl-N-(2.4-dinitrophenyl)-thioharnstoff

N- [(dimethylamino)(4-nitrilo-phenylimino)methyl]-N',N'-dimethyl-N-phenyl-thioharnstoff

N- [(dimethylamino)(4-carboxymethyl-phenylimino)methyl]-N',N'-dimethyl-N-phenyl-thioharnstoff

N- [(dimethylamino)(4-carboxymethyl-phenylimino)methyl]-N',N'-dimethyl-N-(4-carboxymethyl-phenyl)-thioharnstoff

N- [(dimethylamino)(phenylimino)methyl]-N',N'-dimethyl-N-(4-carboxymethyl-phenyl)-thioharnstoff

N- [(dimethylamino)(phenylimino)methyl]-N',N'-dimethyl-N-p-chlorphenyl-thioharnstoff

N- [(dimethylamino)(phenylimino)methyl]-N',N'-dimethyl-N-3.4-dichlorphenyl-thioharnstoff

N-[(dimethylamino)(phenylimino)methyl]-N',N'-dimethyl-N-p-tolyl-thioharnstoff

N-[(dimethylamino)(phenylimino)methyl]-N',N'-dimethyl-N-2.4-dimethylphenyl-thioharnstoff

N-[(dimethylamino)(phenylimino)methyl]-N',N'-dimethyl-N-p-fluorphenyl-thioharnstoff

N-[(dimethylamino)(phenylimino)methyl]-N',N'-dimethyl-N-2.4-dinitrophenyl-thioharnstoff

N-[(dimethylamino)(phenylimino)methyl]-N',N'-dimethyl-N-4-nitrilophenyl-thioharnstoff

N-[(dimethylamino)(α-naphtylimino)methyl]-N',N'-dimethyl-N-phenyl-thioharnstoff

N-[(dimethylamino)(α-naphtylimino)methyl]-N',N'-dimethyl-N-( ' -naphtyl)-thioharnstoff

N-[(dimethylamino)(ß-naphtylimino)methyl]-N',N'-dimethyl-N-ß-naphtyl-thioharnstoff

N-[(dimethylamino)(ß-naphtylimino)methyl]-N',N'-dimethyl-N-phenyl-thioharnstoff

N-[(dimethylamino)( -3'-chloronaphtylimino)methyl]-N',N'-dimethyl-N-phenyl-thioharnstoff

N-[(dimethylamino)( -3'-chloronaphtylimino)methyl]-N',N'-dimethyl-N- -3'-chloronaphtyl-thioharnstoff

N-[(morpholino)(phenylimino)methyl]-N'-pentamethylen-N-phenyl-thioharnstoff

N-[(morpholino)(phenylimino)methyl]-N'-(4'-oxa-pentamethylen)-N-phenyl-thioharnstoff

N-[(piperidino)(phenylimino)methyl]-N'-pentamethylen-N-phenyl-thioharnstoff

N-[(piperidino)(phenylimino)methyl]-N'-(4'-thia-pentamethylen)-N-phenyl-thioharnstoff

N-[(diethyl)(4-bromphenyl)methyl]-N',N'-diethyl-N-phenyl-thioharnstoff

N-[(diethyl)(4-bromphenyl)methyl]-N',N'-diethyl-N-4-bromphenyl-thioharnstoff

N-[(N-Methyl-piperazino)(phenylimino)methyl]-N'-(4'methylaza-pentamethylen)-N-phenyl-thioharnstoff

Die erfindungsgemäßen Wirkstoffe eignen sich zur Bekämpfung von mono- und dikotylen Schadpflanzen in Kulturen des Acker- und Gartenbaus.

Wirkungsbeispiele für dikotyle Schadpflanzen sind Abutilon theophrasti, Amaranthus retroflexus, Capsella bursa-pastoris, Centaurea cyanus, Chenopodium album, Chrysanthemum segetum, Galinsoga parviflora, Galium aparine, Ipomoea purpurea, Lapsana communis, Matricaria inodora, Polygonum persicaria, Portulaca oleracea, Raphanus raphanistrum, Senecio vulgaris, Sinapis arvensis, Solanum nigrum, Stellaria media, Sinapis alba, Sonchus arvensis, Thlaspi arvense, Viola tricolor u.a.

Wirkungsbeispiele für monokotyle Schadpflanzen sind Alopecurus myosuroides, Apera spica-venti, Avena fatua, Digitaria sanguinalis, Poa annua, Setaria viridis, Echinochloa crus-galli, Lolium perenne, Cyperus iria u.a.

Die erfindungsgemäßen Wirkstoffe bewähren sich bei der selektiven Bekämpfung von Schadpflanzen in wichtigen Kulturpflanzungen. Sie verhalten sich z.B. tolerant gegenüber Mais, Sorghum Hirse, Sojabohnen, Buschbohnen, Erbsen, Kartoffeln, Getreide, insbesondere Sommergerste, Wintergerste und Winterweizen, und, besonders hervorzuheben, gegenüber Baumwolle.

Die erfindungsgemäßen Wirkstoffe können allein oder im Gemisch ausgebracht werden. Im allgemeinen werden sie jedoch als Mischungen mit festen oder flüssigen Verdünnungsmitteln oder als Lösungen in festen oder flüssigen Lösungsmitteln verwendet, mit Wirkstoffgehalten von 0,01 bis 95 Gew.%. Die Mischungen bzw. Lösungen werden im allgemeinen als Emulsionskonzentrate, Pasten, Spritzpulver, Granulate oder Mikrokapseln hergestellt.

Emulsionskonzentrate und Pasten enthalten im allgemeinen 10 bis 90 Gew.%, vorzugsweise 15 bis 50 Gew.% Wirkstoff, 2 bis 25 Gew.% Dispergierhilfsstoffe und organische Lösungsmittel und/oder Wasser.

Spritzpulver enthalten meistens 10 bis 80 Gew.%, vorzugsweise 15 bis 70 Gew.% Wirkstoff, 1 bis 10 Gew.% Dispergierhilfsstoffe und 10 bis 89 Gew.% inerte Bestandteile.

Granulate und Mikrokapseln enthalten neben inerten Bestandteilen, Bindemitteln und/oder Überzugsstoffen, 1 bis 10 Gew.%, vorzugsweise 5 bis 10 Gew.% Wirkstoff.

Erfindungsgemäß angewandt werden:

Als Dispergierhilfsstoffe z.B. Alkyl- und Arylsulfonate, Methylzellulose, polymere Sulfonsäuren und deren Salze, Polyalkohole, Fettsäureester, Fettalkoholäther, Fettamine;

als organische Lösungsmittel z.B. Alkohole, wie Ethanol, Butanole, Dimethylformamid, Dimethylsulfoxid, N-Methylpyrrolidon, Aromaten, wie Toluol und Xylole;

als inerte Bestandteile z.B. Kaolin, China-Clay, Talkum, Kalziumcarbonat, hochdisperse Kieselsäure, Kieselgele, Kieselgur, Diatomenerde, Bims, Ziegelsplit, Maisschrot, Verdickungsmittel, wie Stärke und Carboxymethylzellulose;

als Bindemittel z.B. Magnesiumsulfat, Gips, Gummiarabikum, Polyvinylalkohol.

Beispielsweise werden erfindungsgemäße Wirkstoffe zur Verwendung als Herbizide wie folgt formuliert:

Emulsionskonzentrat:

| | |
|---|---|
| 51,4 | Gew.% Wirkstoff |
| 30 | Gew.% Kieselerde |
| 10 | Gew.% hochdisperse Kieselsäure |
| 6 | Gew.% Ligninsulfonat (Zellpech) |
| 2 | Gew.% Polypropylenglykol |
| 0,6 | Gew.% Natriumoleyl-Methyltaurid |

Spritzpulver:

20 Gew.% Wirkstoff

44 Gew.% China-Clay

16 Gew.% hochdisperse Kieselsäure

15 Gew.% Ligninsulfonat (Zellpech)

5 Gew.% Natrium-Alkylnaphtalinsulfonat-Formaldehyd-Kondensat ("Adlox 4862", eingetr. Warenzeichen)

Im allgemeinen werden die Wirkstoffe in Aufwandmengen von 0,25 bis 8 kg/ha, vorzugsweise 1 bis 4 kg/ha, ausgebracht. Die Anwendung als Herbizid kann sowohl auf die Pflanzen (Nachauflaufverfahren) als auch auf vegetationsfreie Bodenoberflächen (Vorauflauf- und Vorsaatverfahren) erfolgen. Der größere herbizide Effekt wird mit einer Bodenapplikation erzielt, vorzugsweise bei Anwendung vor der Aussaat der Kulturpflanzen mit nachfolgender flacher, mechanischer Einarbeitung des ausgebrachten herbiziden Mittels in die obersten Schichten der Bodenoberfläche.

Im folgenden werden Darstellungsmethoden der erfindungsgemäßen Wirkstoffe und ihre herbiziden Eigenschaften anhand von Beispielen erläutert:

Beispiel 1

Herstellung von N-[(dimethylamino)(p-chlorphenylimino)methyl]-N',N'-dimethyl-N-p-chlorphenyl-thioharnstoff

89 g N-(p-Chlorphenyl)-N',N'-dimethyl-thioharnstoff werden in 500 ml Methanol aufgeschlämmt. Zu dieser Suspension wird unter Rühren eine Lösung von 36 g Brom in 90 ml Methanol zugetropft. Die Reaktionstemperatur beträgt 20 °C. Es erfolgt eine sofortige Reaktion unter Schwefelabscheidung. Nachdem das gesamte Brom zugetropft ist, wird die Reaktionsmischung noch 5 Minuten weitergerührt und anschließend kurz bis zum Sieden erhitzt. Danach wird vom Schwefel abfiltriert. Nun wird das Filtrat bis zur alkalischen Reaktion mit 30 %-iger Natronlauge versetzt. Schließlich wird die alkalische Lösung unter Rühren in 1 l Wasser eingetragen.

Das Zielprodukt fällt als farbloser Niederschlag an. Die Ausbeute beträgt 78 g, entsprechend 95 % d. Th. Das aus Ethanol umkristallisierte Produkt besitzt einen Schmelzpunkt von 172 °C.

Beispiel 2

Herstellung von N-[(dimethylamino)(p-tolylimino)methyl]-N',N'-dimethyl-N-p-tolyl-thioharnstoff

121 g N-(p-tolyl)-N',N'-dimethyl-thioharnstoff werden in 750 ml Ethanol in einem Dreihalskolben mit Rüher und Gaseinlaßteil aufgeschlämmt. Es werden nun unter Rühren der Suspension, über ein Rotameter kontrolliert, 24 g Chlorgas zugeführt. Es tritt sofortige Reaktion unter Schwefelabscheidung ein. Die Reaktionstemperatur beträgt 25 °C. Nach beendeter Chlorzugabe wird bis zum Sieden erhitzt. Danach wird der ausgefallene Schwefel abfiltriert, das Filtrat, wie in Beispiel 1 beschrieben, alkalisch gestellt und schließlich in 1,5 l Wasser eingetragen.

Es fällt ein farbloser Niederschlag an Zielprodukt an. Die Ausbeute beträgt 106 g, entsprechend 96 % d. Th. Das Zielprodukt besitzt nach Umkristallisieren in Cyclohexan einen Schmelzpunkt von 167 °C.

Beispiel 3

Herstellung von N-[(dimethylamino)(phenylimino)methyl]-N',N'-dimethyl-N-phenyl-thioharnstoff

a) Herstellung von N-phenyl-N',N'-dimethyl-chlorformamidin (I)

Einer Lösung von 55 g Phenylisocyanat in 500 ml Benzol werden 23 g Dimethylamin zugetropft. Es erfolgt sofortige Bildung von N-phenyl-N',N'-dimethylharnstoff. Anschließend werden 100 ml Benzol abdestilliert, um damit gleichzeitig geringe Mengen nicht umgesetzten Dimethylamins abzutreiben. Der verbleibenden Reaktionsmischung werden nun portionsweise 97 g $PCl_5$ zugegeben. Es tritt sofortige Reaktion unter kräftiger HCl-Entwicklung

ein. Nach beendeter $PCl_5$-Zugabe wird zum Sieden erhitzt und ca. 15 Minuten am Rückfluß gekocht. Es bilden sich zwei Schichten. Schließlich wird Benzol und entstandenes $POCl_3$ abgezogen. Das gewünschte Chlorformamidin (I) wird nun bei 5 Torr rektifiziert.

b) Umsetzung von (I) mit N-phenyl-N',N'-dimethyl-thioharnstoff (II)

Es werden die Lösungen von 15 g (I) in 15 ml Chloroform und 15 g (II) in 30 ml Chloroform vereinigt. Die Mischung wird 24 Stunden bei einer Temperatur von 23 °C stehengelassen. Danach wird das Chloroform abgezogen und der Rückstand in wenig Methanol gelöst. Danach wird die Lösung mit 30 %-iger Natronlauge bis zu alkalischen Reaktion versetzt. Schließlich wird die alkalische Lösung unter kräftigem Rühren in 700 ml Wasser eingetragen. Das Zielprodukt fällt als farbloser Niederschlag an. Nach Umkristallisieren in einem Methanol/Wasser-Gemisch werden in einer Ausbeute von 20 g farblose Kristalle vom Schmelzpunkt 118 °C erhalten.

Beispiel 4

Herstellung von N-[(dimethylamino)(phenylimino)methyl]-N',N'-dimethyl-N-p-chlorphenyl-thioharnstoff

Es werden die Lösungen vereinigt von 15 g des gemäß Beispiel 3 a) hergestellten Formamidins in 15 ml Chloroform und 18 g N-p-chlorphenyl-N',N'-dimethyl-thioharnstoff in 30 ml Chloroform. Nach 24-stündigem Stehenlassen bei 25 °C wird das Chloroform abgezogen, der Rückstand in wenig Methanol gelöst, die Lösung mit 30 %-iger Natronlauge alkalisch gestellt und die alkalische Lösung unter Rühren in 700 ml Wasser eingetragen. Der Niederschlag wird abfiltriert und aus Ethanol umkristallisiert. Das Zielprodukt fällt in Form farbloser Kristalle in einer Ausbeute von 21 g an.

Beispiel 5

Herbizide Wirksamkeit von N-[(dimethylamino)(phenylimino)methyl]-N',N'-dimethyl-N-phenyl-thioharnstoff

Die herbizide Wirksamkeit der erfindungsgemäßen Verbindung bei einmaliger Anwendung wurde in Gewächshausversuchen getestet. Im dargestellten Test wurden zunächst die Samenkörner der Kulturpflanzen und Unkräuter in mineralischem, humusarmem Ackerboden ausgesät und leicht mit Bodenpartikeln bedeckt. Unmittelbar nach der Aussaat, auf jeden Fall vor dem Aufkeimen der im Boden befindlichen Samenkörner, wurde der erfindungsgemäße Wirkstoff in Form von Spritzpulvern oder Emulsionskonzentraten auf die vegetationsfreie Bodenoberfläche gleichmäßig aufgespritzt. 4 Wochen nach der Behandlung wurden die Pflanzen abschließend auf Schädigung bzw. Abtötung bonitiert, wobei inzwischen unbehandelt herangewachsene Kontrollpflanzen als Bezug dienten.

Tabelle 1

Wirksamkeit in % bei Anwendung von 0,25 bis 3 kg/ha Aktivsubstanz im Vorauflaufverfahren

| | kg Aktivsubstanz/ha | | | | |
|---|---|---|---|---|---|
| | 0,25 | 0,5 | 1,0 | 2,0 | 3,0 |
| Nutzpflanzen | | | | | |
| Mais | 0 | 0 | 0 | 0 | 15 |
| Sorghum-Hirse | 0 | 0 | 0 | 0 | 30 |
| Sojabohnen | 0 | 0 | 10 | 30 | 45 |
| Buschbohnen | 0 | 0 | 0 | 10 | 40 |
| Baumwolle | 0 | 0 | 0 | 10 | 30 |
| Dikotyle Unkräuter | | | | | |
| Abutilon theophrasti | 95 | 100 | 100 | 100 | - |
| Amaranthus retroflexus | 80 | 95 | 100 | 100 | - |
| Capsella bursa-pastoris | 95 | 100 | 100 | 100 | - |
| Chenopodium album | 85 | 100 | 100 | 100 | - |
| Chrysanthemum segetum | 90 | 100 | 100 | 100 | - |

| | kg Aktivsubstanz/ha | | | | |
|---|---|---|---|---|---|
| | 0,25 | 0,5 | 1,0 | 2,0 | 3,0 |
| Galinsoga parviflora | 90 | 100 | 100 | 100 | - |
| Ipomoea purpurea | 65 | 80 | 100 | 100 | - |
| Matricaria inodora | 100 | 100 | 100 | 100 | - |
| Polygonum persicaria | 75 | 90 | 100 | 100 | - |
| Portulaca oleracea | 100 | 100 | 100 | 100 | - |
| Raphanus raphanistrum | 95 | 100 | 100 | 100 | - |
| Stellaria media | 75 | 85 | 95 | 100 | - |
| Senecio vulgaris | 70 | 95 | 100 | 100 | - |
| Sinapis alba | 60 | 70 | 95 | 100 | - |
| Sonchus arvensis | 100 | 100 | 100 | 100 | - |
| Thlaspi arvense | 95 | 100 | 100 | 100 | - |
| Ungräser | | | | | |
| Apera spica-venti | 100 | 100 | 100 | 100 | - |
| Alopecurus myosuroides | 60 | 80 | 95 | 100 | - |
| Avena fatua | 60 | 70 | 85 | 100 | - |
| Digitaria sanguinalis | 65 | 75 | 75 | 100 | - |
| Poa annua | 100 | 100 | 100 | 100 | - |
| Setaria viridis | 65 | 80 | 95 | 100 | - |

Beispiel 6

Herbizide Wirksamkeit von N-[(dimethylamino)(3.4-dichlorphenylimino)methyl]-N',N'-dimethyl-N-3.4-dichlorphenyl-thioharnstoff

Der erfindungsgemäße Wirkstoff wird analog Beispiel 5 im Vorauflaufverfahren getestet

Tabelle 2

Wirksamkeit in % bei Aufwandmengen von 0,5 bis 4 kg/ha Aktivsubstanz im Vorauflaufverfahren

| | kg/ha Aktivsubstanz | | | |
|---|---|---|---|---|
| | 0,5 | 1,0 | 2,0 | 4,0 |
| Nutzpflanzen | | | | |
| Mais | 0 | 0 | 0 | 10 |
| Sorghum-Hirse | 0 | 0 | 0 | 20 |
| Buschbohnen | 0 | 0 | 10 | 60 |
| Sojabohnen | 0 | 0 | 0 | 30 |
| Erbsen | 0 | 0 | 10 | 40 |
| Baumwolle | 0 | 0 | 0 | 30 |
| Dikotyle Unkräuter | | | | |
| Amaranthus retroflexus | 75 | 90 | 100 | 100 |
| Capsella bursa-pastoris | 100 | 100 | 100 | 100 |
| Chenopodium album | 100 | 100 | 100 | 100 |
| Chrysanthemum segetum | 60 | 85 | 100 | 100 |
| Galinsoga parviflora | 85 | 100 | 100 | 100 |
| Matricaria inodora | 90 | 100 | 100 | 100 |
| Polygonum persicaria | 50 | 80 | 90 | 100 |
| Sinapis alba | 50 | 75 | 90 | 95 |
| Stellaria media | 80 | 95 | 100 | 100 |
| Solanum nigrum | 60 | 100 | 100 | 100 |
| Ungräser | | | | |
| Alopecurus myosuroides | 50 | 70 | 80 | 100 |
| Apera spica-venti | 80 | 100 | 100 | 100 |
| Avena fatua | 50 | 70 | 80 | 85 |
| Digitaria sanguinalis | 40 | 80 | 100 | 100 |
| Poa annua | 85 | 100 | 100 | 100 |
| Setaria viridis | 50 | 85 | 100 | 100 |

Beispiel 7

Herbizide Wirksamkeit von N-[(dimethylamino)(p-chlorphenylimino)methyl]-N',N'-dimethyl-p-chlorphenyl-thioharnstoff

Der erfindungsgemäße Wirkstoff wird analog Beispiel 5 im Vorauflaufverfahren getestet.

Tabelle 3

Wirksamkeit in % bei Aufwandmengen von 0,5 bis 4 kg/ha Aktivsubstanz im Vorauflaufverfahren

| | kg/ha Aktivsubstanz | | | |
|---|---|---|---|---|
| | 0,5 | 1,0 | 2,0 | 4,0 |
| Nutzpflanzen | | | | |
| Mais | 0 | 0 | 0 | 10 |
| Sorghum-Hirse | 0 | 0 | 10 | 50 |
| Buschbohnen | 0 | 0 | 0 | 10 |
| Sojabohnen | 0 | 30 | 50 | 70 |
| Erbsen | 0 | 10 | 15 | 30 |
| Baumwolle | 0 | 0 | 0 | 10 |
| Dikotyle Unkräuter | | | | |
| Amaranthus retroflexus | 85 | 100 | 100 | 100 |
| Capsella bursa-pastoris | 100 | 100 | 100 | 100 |
| Chenopodium album | 100 | 100 | 100 | 100 |
| Chrysanthemum segetum | 85 | 100 | 100 | 100 |
| Galinsoga parviflora | 100 | 100 | 100 | 100 |
| Matricaria inodora | 100 | 100 | 100 | 100 |
| Polygonum persicaria | 70 | 95 | 100 | 100 |
| Sinapis alba | 75 | 100 | 100 | 100 |
| Stellaria media | 95 | 100 | 100 | 100 |
| Solanum nigrum | 80 | 100 | 100 | 100 |
| Ungräser | | | | |
| Alopecurus myosuroides | 75 | 95 | 100 | 100 |
| Apera spica-venti | 100 | 100 | 100 | 100 |
| Avena fatua | 70 | 95 | 100 | 100 |
| Digitaria sanguinalis | 65 | 90 | 100 | 100 |
| Poa annua | 100 | 100 | 100 | 100 |
| Setaria viridis | 60 | 80 | 100 | 100 |

Beispiel 8

Herbizide Wirksamkeit von N-[(dimethylamino)(m-tolylimino)methyl]-N',N'-dimethyl-N-m-tolyl-thioharnstoff

Das erfindungsgemäße Herbizid wird analog Beispiel 5 im Vorauflaufverfahren getestet.

Tabelle 4

Wirksamkeit in % bei Anwendung von 0,5 bis 4 kg/ha Aktivsubstanz im Vorauflaufverfahren

| | kg/ha Aktivsubstanz | | | |
|---|---|---|---|---|
| | 0,5 | 1,0 | 2,0 | 4,0 |
| Nutzpflanzen | | | | |
| Mais | 0 | 0 | 0 | 0 |
| Sorghum-Hirse | 0 | 0 | 0 | 20 |
| Dikotyle Unkräuter | | | | |
| Amaranthus retroflexus | 0 | 30 | 40 | 70 |
| Capsella bursa-pastoris | 85 | 90 | 100 | 100 |
| Chenopodium album | 75 | 80 | 95 | 100 |
| Chrysanthemum segetum | 60 | 70 | 100 | 100 |
| Galinsoga parviflora | 70 | 85 | 100 | 100 |
| Matricaria inodora | 75 | 95 | 100 | 100 |
| Polygonum persicaria | 60 | 80 | 100 | 100 |
| Sinapis alba | 70 | 75 | 80 | 100 |
| Stellaria media | 30 | 65 | 95 | 100 |
| Solanum nigrum | 0 | 40 | 75 | 80 |
| Ungräser | | | | |
| Alopecurus myosuroides | 50 | 75 | 85 | 95 |
| Apera spica-venti | 95 | 95 | 100 | 100 |
| Avena fatua | 60 | 70 | 85 | 100 |
| Digitaria sanguinalis | 20 | 60 | 75 | 100 |
| Poa annua | 90 | 100 | 100 | 100 |
| Setaria viridis | 80 | 95 | 100 | 100 |

0056446

Beispiel 9

Herbizide Wirksamkeit von N-[(dimethylamino)(p-fluorphenyl)methyl]-N',N'-dimethyl-N-p-fluorphenyl-thioharnstoff

Der erfindungsgemäße Wirkstoff wird analog Beispiel 5 im Vorauflaufverfahren getestet

Tabelle 5

Wirksamkeit in % bei Aufwandmengen von 0,5 bis 4 kg/ha Aktivsubstanz im Vorauflaufverfahren

| | kg/ha Aktivsubstanz | | | |
|---|---|---|---|---|
| | 0,5 | 1,0 | 2,0 | 4,0 |
| Kulturpflanzen | | | | |
| Mais | 0 | 0 | 0 | 10 |
| Sorghum-Hirse | 0 | 0 | 0 | 20 |
| Baumwolle | 0 | 0 | 10 | 30 |
| Sojabohnen | 0 | 0 | 10 | 50 |
| Buschbohnen | 0 | 0 | 10 | 30 |
| Dikotyle Unkräuter | | | | |
| Amaranthus retroflexus | 100 | 100 | 100 | 100 |
| Centaurea cyanus | 20 | 70 | 85 | 100 |
| Chrysanthemum segetum | 95 | 100 | 100 | 100 |
| Galinsoga parviflora | 95 | 100 | 100 | 100 |
| Matricaria inodora | 100 | 100 | 100 | 100 |
| Polygonum persicaria | 65 | 80 | 90 | 100 |
| Senecio vulgaris | 75 | 85 | 100 | 100 |
| Sinapis arvensis | 60 | 85 | 95 | 100 |
| Solanum nigrum | 85 | 95 | 95 | 100 |
| Stellaria media | 75 | 85 | 95 | 100 |
| Ungräser | | | | |
| Alopecurus myosuroides | 75 | 80 | 95 | 95 |
| Apera spica-venti | 95 | 100 | 100 | 100 |
| Digitaria sanguinalis | 95 | 100 | 100 | 100 |
| Poa annua | 85 | 85 | 95 | 100 |
| Setaria viridis | 80 | 90 | 100 | 100 |

Beispiel 10

Herbizide Wirksamkeit von N-[(dimethylamino)(3-chlor-4-fluor-phenyl)methyl]-N',N'-dimethyl-N-(3-chlor-4-fluor-phenyl)-thioharnstoff

Das erfindungsgemäße Herbizid wird analog Beispiel 5 getestet.

Tabelle 6

Wirksamkeit in % bei Anwendung von 0,5 bis 4 kg/ha Aktivsubstanz im Vorauflaufverfahren

| | kg/ha Aktivsubstanz | | | |
|---|---|---|---|---|
| | 0,5 | 1,0 | 2,0 | 4,0 |
| Kulturpflanzen | | | | |
| Mais | 0 | 0 | 10 | 20 |
| Sorghum-Hirse | 0 | 10 | 30 | 50 |
| Sojabohnen | 0 | 0 | 10 | 60 |
| Buschbohnen | 0 | 0 | 30 | 50 |
| Ackerbohnen | 0 | 0 | 40 | 70 |
| Baumwolle | 0 | 0 | 10 | 30 |
| Dikotyle Unkräuter | | | | |
| Amaranthus retroflexus | 100 | 100 | 100 | 100 |
| Chrysanthemum segetum | 100 | 100 | 100 | 100 |
| Galinsoga parviflora | 65 | 95 | 100 | 100 |
| Matricaria inodora | 75 | 90 | 100 | 100 |
| Polygonum persicaria | 95 | 95 | 100 | 100 |
| Senecio vulgaris | 95 | 100 | 100 | 100 |
| Sinapis arvensis | 85 | 90 | 95 | 100 |
| Solanum nigrum | 85 | 95 | 100 | 100 |
| Stellaria media | 80 | 95 | 100 | 100 |
| Ungräser | | | | |
| Alopecurus myosuroides | 75 | 85 | 95 | 100 |
| Apera spica-venti | 100 | 100 | 100 | 100 |
| Digitaria sanguinalis | 95 | 100 | 100 | 100 |
| Poa annua | 85 | 95 | 95 | 100 |
| Setaria viridis | 100 | 100 | 100 | 100 |

Vergleichsbeispiel 1

Zum Vergleich werden das zu den Harnstoff-Derivaten gehörende Handelsherbizid Afalon mit dem Wirkstoff Linuron [3-(3.4-dichlorphenyl)-1-methoxy-1-methyl-harnstoff] und das zur Gruppe der Acetanilide zuzurechnende Handelsprodukt Lasso mit dem Wirkstoff Alachlor [2-chlor-N-(2.6-diethylphenyl)-N-(methoxymethyl)acetamid] getestet. Im übrigen wird wie gemäß Beispiel 5 beschrieben verfahren.

Tabelle 7

Wirksamkeit in % bei Aufwandmengen von 0,25 bis 3,0 kg/ha Aktivsubstanz im Vorauflaufverfahren

| | LINURON | | | | | ALACHLOR | | | | |
|---|---|---|---|---|---|---|---|---|---|---|
| | 0,25 | 0,5 | 1,0 | 2,0 | 3,0 | 0,25 | 0,5 | 1,0 | 2,0 | 3,0 |
| Nutzpflanzen | | | | | | | | | | |
| Mais | 0 | 10 | 15 | 40 | 70 | 0 | 0 | 0 | 0 | 10 |
| Sorghum-Hirse | 10 | 10 | 30 | 80 | 100 | 50 | 80 | 95 | 100 | 100 |
| Sojabohnen | 0 | 10 | 50 | 85 | 100 | 0 | 0 | 0 | 0 | 10 |
| Buschbohnen | 0 | 40 | 50 | 70 | 95 | 0 | 0 | 0 | 30 | 60 |
| Baumwolle | 0 | 30 | 70 | 80 | 100 | 0 | 10 | 20 | 30 | 50 |
| Dikotyle Unkräuter | | | | | | | | | | |
| Abutilon theophrasti | 90 | 100 | 100 | | | 0 | 0 | 0 | 0 | 0 |
| Amaranthus retroflexus | 95 | 100 | 100 | | | 75 | 90 | 100 | 100 | 100 |
| Capsella bursa-pastoris | 95 | 100 | 100 | | | - | - | - | - | - |
| Chenopodium album | 80 | 95 | 100 | | | 0 | 0 | 10 | 30 | 75 |
| Chrysanthemum segetum | 85 | 90 | 100 | | | 0 | 0 | 10 | 70 | 80 |
| Galinsoga parviflora | 95 | 100 | 100 | | | 0 | 10 | 65 | 90 | 100 |
| Ipomoea purpurea | 10 | 50 | 75 | 95 | 100 | 0 | 0 | 0 | 0 | 10 |
| Matricaria inodora | 90 | 95 | 100 | | | 0 | 10 | 70 | 90 | 90 |
| Polygonum persicaria | 80 | 90 | 100 | | | - | - | - | - | - |
| Portulaca oleracea | 95 | 100 | 100 | | | 90 | 90 | 100 | 100 | 100 |
| Raphanus raphanistrum | 50 | 60 | 70 | 80 | 80 | 0 | 0 | 0 | 0 | 10 |

| | LINURON | | | | | ALACHLOR | | | | |
|---|---|---|---|---|---|---|---|---|---|---|
| | 0,25 | 0,5 | 1,0 | 2,0 | 3,0 | 0,25 | 0,5 | 1,0 | 2,0 | 3,0 |
| Stellaria media | 80 | 90 | 100 | | | 0 | 0 | 20 | 60 | 85 |
| Thlaspi arvense | 100 | 100 | | | | 0 | 0 | 0 | 0 | 15 |
| Senecio vulgaris | 40 | 60 | 75 | 95 | 100 | 0 | 0 | 0 | 10 | 70 |
| Sinapis alba | 70 | 90 | 95 | 100 | 100 | 0 | 0 | 0 | 0 | 25 |
| Sonchus arvensis | 90 | 90 | 100 | 100 | 100 | 0 | 10 | 50 | 80 | 90 |
| Ungräser | | | | | | | | | | |
| Apera spica-venti | 90 | 95 | 100 | | | 60 | 75 | 80 | 95 | 95 |
| Alopecurus myosuroides | 30 | 55 | 80 | 95 | 100 | 10 | 30 | 80 | 95 | 100 |
| Avena fatua | 10 | 60 | 85 | 90 | 95 | 10 | 20 | 65 | 65 | 80 |
| Digitaria sanguinalis | 80 | 90 | 95 | 100 | 100 | 80 | 95 | 100 | | |
| Poa annua | 95 | 95 | 100 | | | 50 | 85 | 95 | 100 | 100 |
| Setaria viridis | 65 | 75 | 90 | 95 | 100 | 85 | 95 | 95 | 100 | 100 |

Der Vergleich der Ergebnisse gemäß den Beispielen 5 bis 9 mit den Daten gemäß Vergleichsbeispiel 1 verdeutlicht die verbesserte Nutzpflanzenverträglichkeit der erfindungsgemäßen Wirkstoffe, insbesondere gegenüber dem Standardherbizid Linuron. Auffällig ist weiterhin die wesentlich höhere herbizide Wirksamkeit der erfindungsgemäßen Substanzen im Vergleich zum praxisüblichen Wirkstoff Alachlor gegenüber dikotylen Unkräutern.

Beispiel 11

Herbizide Wirksamkeit von N-[(dimethylamino)(phenylimino)methyl]-N',N'-dimethyl-N-phenyl-thioharnstoff im Freilandversuch

Die landwirtschaftliche Praxis verfährt im wesentlichen nach der gemäß Beispiel 5 beschriebenen Methode, nach der die Samenkörner der Nutzpflanzen in die oberste Schicht einer vegetationsfreien Oberfläche eines maschinell bearbeiteten Ackerbodens zur Keimung abgelegt werden.

Die Samen der Schadpflanzen sind in unterschiedlicher Zusammensetzung in jedem Ackerboden stets vorhanden und keimen mit den ausgesäten Nutzpflanzen automatisch auf.

Der erfindungsgemäße Wirkstoff wurde in den Freilandversuchen nach der gemäß Beispiel 5 beschriebenen Methode appliziert. Je nach Entwicklungsdauer der entsprechenden Kulturpflanze wurde nach 4 bis 8 Wochen abschließend auf Schädigung bzw. Abtötung der Pflanzen bonitiert, wobei inzwischen unbehandelt gebliebene Kontrollparzellen als Bezug dienten.

Tabelle 8

Wirksamkeit in % bei Aufwandmengen von 0,5 bis 4 kg/ha Wirksubstanz im Vorauflaufverfahren

| | erfindungsgem. Wirkstoff | | | |
|---|---|---|---|---|
| | 0,5 | 1,0 | 2,0 | 4,0 |
| Nutzpflanzen | | | | |
| Mais | 0 | 0 | 0 | 0 |
| Sorghum-Hirse | 0 | 0 | 0 | 10 |
| Sojabohnen | 0 | 0 | 0 | 10 |
| Baumwolle | 0 | 0 | 0 | 20 |
| Buschbohnen | 0 | 0 | 0 | 10 |
| Dikotyle Unkräuter | | | | |
| Abutilon theophrasti | 60 | 75 | 95 | 100 |
| Amaranthus retroflexus | 85 | 100 | 100 | 100 |
| Anthemis arvensis | 100 | 100 | 100 | 100 |
| Chenopodium album | 100 | 100 | 100 | 100 |
| Galinsoga parviflora | 95 | 100 | 100 | 100 |
| Portulaca aleracea | 100 | 100 | 100 | 100 |
| Raphanus raphanistrum | 95 | 100 | 100 | 100 |
| Sinapis alba | 90 | 95 | 100 | 100 |
| Sonchus arvensis | 100 | 100 | 100 | 100 |
| Thlaspi arvense | 95 | 100 | 100 | 100 |

Die in Gewächshausprüfungen gefundene hohe herbizide Wirksamkeit der erfindungsgemäßen Wirkstoffe konnte in Freilandversuchen unter Praxisbedingungen bestätigt werden, was am Beispiel des obengenannten Wirkstoffs eindrucksvoll demonstriert wird.

Vergleichsbeispiel 2

Zum Vergleich werden die bereits gemäß Vergleichsbeispiel 1 beschriebenen bekannten Herbizidwirkstoffe Linuron und Alachlor unter Freilandbedingungen analog Beispiel 11 untersucht.

Tabelle 9

Wirksamkeit in % bei Aufwandmengen von 0,5 bis 4 kg/ha Aktivsubstanz im Vorauflaufverfahren

| | LINURON | | | | ALACHLOR | | | |
|---|---|---|---|---|---|---|---|---|
| | 0,5 | 1,0 | 2,0 | 4,0 | 0,5 | 1,0 | 2,0 | 4,0 |
| Nutzpflanzen | | | | | | | | |
| Mais | 0 | 10 | 20 | 100 | 0 | 0 | 0 | 10 |
| Sorghum-Hirse | 10 | 20 | 70 | 100 | 30 | 90 | 100 | 100 |
| Sojabohnen | 0 | 10 | 30 | 100 | 0 | 0 | 0 | 0 |
| Baumwolle | 10 | 30 | 100 | 100 | 0 | 10 | 30 | 50 |
| Buschbohnen | 10 | 30 | 80 | 100 | 0 | 0 | 20 | 60 |
| Dikotyle Unkräuter | | | | | | | | |
| Abutilon theophrasti | 75 | 90 | 100 | 100 | 0 | 0 | 0 | 0 |
| Amaranthus retroflexus | 60 | 85 | 100 | 100 | 50 | 75 | 100 | 100 |
| Anthemis arvensis | - | - | - | - | 60 | 70 | 85 | 100 |
| Chenopodium album | 85 | 90 | 100 | 100 | 0 | 10 | 70 | 80 |
| Galinsoga parviflora | 95 | 100 | 100 | 100 | 70 | 85 | 100 | 100 |
| Portulaca aleracea | 80 | 100 | 100 | 100 | 80 | 90 | 95 | 100 |
| Raphanus raphanistrum | 90 | 100 | 100 | 100 | 0 | 0 | 0 | 0 |
| Sinapis alba | 85 | 95 | 100 | 100 | 0 | 0 | 10 | 30 |
| Sonchus arvensis | 75 | 80 | 90 | 100 | 65 | 80 | 100 | 100 |
| Thlaspi arvense | 80 | 100 | 100 | 100 | 0 | 0 | 10 | 20 |

Der Vergleich der Ergebnisse von Beispiel 9 mit Vergleichsbeispiel 2 bestätigt die bereits in den Gewächshaustests gefundene verbesserte Nutzpflanzenverträglichkeit der erfindungsgemäßen Wirkstoffe gegenüber dem Standardwirkstoff Linuron sowie ihre wesentlich höhere herbizide Wirksamkeit im Vergleich zu Alachlor gegenüber dikotylen Unkräutern.

## Beispiel 12

Herbizide Wirksamkeit von N-[(dimethylamino)(phenylimidino)methyl]-N',N'-dimethyl-N-phenyl-thioharnstoff im Nachauflaufverfahren

Die Einsatzmöglichkeiten des erfindungsgemäßen Wirkstoffs im Nachauflaufverfahren (Applikation auf die Blätter der Pflanzen) wurde in einer weiteren Versuchsreihe im Gewächshaus untersucht.

Bei diesem Verfahren wurde der Wirkstoff auf junge Pflanzen gespritzt, die ein bestimmtes Wachstumsstadium erreicht hatten: bei zweikeimblättrigen (dikotylen) Pflanzen war neben den primären Keimblättern das erste, echte Blattpaar entwickelt, bei einkeimblättrigen (monokotylen) Pflanzen waren mindestens zwei Blätter ausgebildet.

Die behandelten Pflanzen wurden das letztemal 14 Tage nach der Spritzung auf Schädigung bzw. Abtötung bonitiert.

### Tabelle 10

Herbizide Wirksamkeit in % von 1 bis 4 kg/ha Aktivsubstanz im Nachauflaufverfahren

| | 1,0 | 2,0 | 4,0 |
|---|---|---|---|
| Nutzpflanzen | | | |
| Sommergerste | 0 | 0 | 10 |
| Mais | 0 | 0 | 0 |
| Sorghum-Hirse | 0 | 0 | 0 |
| Wintergerste | 0 | 0 | 10 |
| Winterweizen | 0 | 0 | 20 |

| | 1,0 | 2,0 | 4,0 |
|---|---|---|---|
| Dikotyle Unkräuter | | | |
| Amaranthus retroflexus | 85 | 90 | 100 |
| Capsella bursa-pastoris | 100 | 100 | 100 |
| Chenopodium album | 100 | 100 | 100 |
| Chrysanthemum segetum | 40 | 60 | 85 |
| Galium aparine | 75 | 100 | 100 |
| Lapsana communis | 85 | 95 | 100 |
| Matricaria inodora | 65 | 80 | 100 |
| Polygonum persicaria | 100 | 100 | 100 |
| Sinapis alba | 75 | 90 | 100 |
| Solanmum nigrum | 70 | 85 | 100 |
| Stellaria media | 50 | 60 | 85 |
| Viola tricolor | 60 | 80 | 90 |
| Ungräser | | | |
| Apera spica-venti | 80 | 95 | 100 |
| Digitaria sanguinalis | 40 | 50 | 75 |
| Poa annua | 95 | 100 | 100 |
| Setaria viridis | 95 | 100 | 100 |

## Beispiel 13

Herbizide Wirksamkeit von N-[(dimethylamino)(3.4-dichlorphenylimino)methyl]-N',N'-dimethyl-N-3.4-dichlorphenyl-thioharnstoff im Nachauflaufverfahren

Der erfindungsgemäße Wirkstoff wird analog Beispiel 12 im Nachauflaufverfahren getestet.

Tabelle 11

Wirksamkeit in % bei Aufwandmengen von 0,25 bis 4 kg/ha Aktivsubstanz

| | 0,25 | 0,5 | 1,0 | 2,0 | 4,0 |
|---|---|---|---|---|---|
| Nutzpflanzen | | | | | |
| Sommergerste | 0 | 0 | 0 | 0 | 0 |
| Mais | 0 | 0 | 0 | 0 | 0 |
| Sorghum-Hirse | 0 | 0 | 0 | 0 | 0 |
| Wintergerste | 0 | 0 | 0 | 0 | 0 |
| Winterweizen | 0 | 0 | 0 | 0 | 10 |
| Dikotyle Unkräuter | | | | | |
| Amaranthus retroflexus | 100 | 100 | 100 | 100 | 100 |
| Capsella bursa-pastoris | 100 | 100 | 100 | 100 | 100 |
| Chenopodium album | 90 | 100 | 100 | 100 | 100 |
| Chrysanthemum segetum | 10 | 50 | 95 | 100 | 100 |
| Galium aparine | 70 | 85 | 90 | 100 | 100 |
| Lapsana communis | 70 | 85 | 85 | 100 | 100 |
| Matricaria inodora | 10 | 65 | 80 | 100 | 100 |
| Polygonum persicaria | 75 | 95 | 100 | 100 | 100 |
| Sinapis alba | 60 | 60 | 80 | 95 | 100 |
| Solanum nigrum | 80 | 100 | 100 | 100 | 100 |
| Stellaria media | 50 | 70 | 100 | 100 | 100 |
| Viola tricolor | 40 | 80 | 100 | 100 | 100 |
| Ungräser | | | | | |
| Apera spica-venti | 40 | 60 | 85 | 95 | 95 |
| Digitaria sanguinalis | 10 | 50 | 80 | 90 | 100 |
| Poa annua | 0 | 40 | 60 | 80 | 85 |
| Setaria viridis | 60 | 80 | 100 | 100 | 100 |

Beispiel 14

Herbizide Wirksamkeit von N- [(dimethylamino)(p-chlorphenylimino) methyl ]-N',N'-dimethyl-p-chlorphenyl-thioharnstoff im Nachauflaufverfahren

Das erfindungsgemäße Herbizid wird analog Beispiel 12 im Nachauflaufverfahren getestet.

Tabelle 12

Wirksamkeit in % bei Anwendung von 1 bis 4 kg/ha Aktivsubstanz im Nachauflaufverfahren

| | 1,0 | 2,0 | 4,0 |
|---|---|---|---|
| Nutzpflanzen | | | |
| Sommergerste | 0 | 0 | 0 |
| Mais | 0 | 0 | 0 |
| Sorghum-Hirse | 0 | 0 | 0 |
| Wintergerste | 0 | 0 | 0 |
| Winterweizen | 0 | 0 | 0 |
| Dikotyle Unkräuter | | | |
| Amaranthus retroflexus | 40 | 50 | 75 |
| Capsella bursa-pastoris | 90 | 95 | 100 |
| Chenopodium album | 85 | 95 | 100 |
| Chrysanthemum segetum | 50 | 70 | 100 |
| Galium aparine | 10 | 65 | 100 |
| Lapsana communis | 0 | 10 | 60 |
| Matricaria inodora | 30 | 70 | 70 |
| Polygonum persicaria | 60 | 90 | 95 |
| Sinapis alba | 0 | 10 | 50 |
| Solanum nigrum | 40 | 65 | 75 |
| Stellaria media | 60 | 75 | 100 |
| Viola tricolor | 10 | 50 | 65 |

| | 1,0 | 2,0 | 4,0 |
|---|---|---|---|
| Ungräser | | | |
| Apera spica-venti | 50 | 50 | 70 |
| Digitaria sanguinalis | 60 | 80 | 95 |
| Poa annua | 70 | 90 | 100 |
| Setaria viridis | 45 | 85 | 100 |

Beispiel 15

Herbizide Wirksamkeit von N-[(dimethylamino)(m-tolylimino)methyl]-N',N'-dimethyl-N-m-tolyl-thioharnstoff im Nachauflaufverfahren

Die herbizide Wirksamkeit des erfindungsgemäßen Wirkstoffs wird im Nachauflaufverfahren analog Beispiel 12 getestet.

Tabelle 13

Wirksamkeit in % bei Anwendung von 1 bis 4 kg/ha Aktivsubstanz im Nachauflaufverfahren

| | 1,0 | 2,0 | 4,0 |
|---|---|---|---|
| Nutzpflanzen | | | |
| Sommergerste | 0 | 0 | 20 |
| Mais | 0 | 0 | 0 |
| Sorghum-Hirse | 0 | 0 | 0 |
| Wintergerste | 0 | 0 | 0 |
| Winterweizen | 0 | 0 | 10 |
| Dikotyle Unkräuter | | | |
| Amaranthus retroflexus | 60 | 90 | 90 |
| Capsella bursa-pastoris | 85 | 100 | 100 |
| Chenopodium album | 80 | 100 | 100 |
| Chrysanthemum segetum | 70 | 75 | 100 |
| Galium aparine | 65 | 80 | 95 |
| Lapsana communis | 10 | 60 | 95 |
| Matricaria inodora | 20 | 50 | 85 |

| | 1,0 | 2,0 | 4,0 |
|---|---|---|---|
| Polygonum persicaria | 70 | 80 | 100 |
| Sinapis alba | 55 | 70 | 95 |
| Solanum nigrum | 60 | 75 | 100 |
| Stellaria media | 10 | 50 | 70 |
| Viola tricolor | 10 | 50 | 70 |
| Ungräser | | | |
| Apera spica-venti | 50 | 75 | 85 |
| Digitaria sanguinalis | 40 | 70 | 95 |
| Poa annua | 60 | 60 | 85 |
| Setaria viridis | 10 | 40 | 75 |

Vergleichsbeispiel 3

Zum Vergleich wurde das auch im Nachauflaufverfahren eingesetzte Handelsprodukt Afalon mit dem Wirkstoff Linuron getestet. Im übrigen wurde wie unter Beispiel 12 beschrieben verfahren.

Tabelle 14

Wirksamkeit in % bei Anwendung von 0,25 bis 4 kg/ha Aktivsubstanz im Nachauflaufverfahren

| | 0,25 | 0,5 | 1,0 | 2,0 | 4,0 |
|---|---|---|---|---|---|
| Nutzpflanzen | | | | | |
| Sommergerste | 30 | 85 | 95 | 100 | 100 |
| Wintergerste | 10 | 60 | 90 | 100 | 100 |
| Winterweizen | 25 | 75 | 95 | 100 | 100 |
| Mais | 10 | 30 | 50 | 75 | 100 |
| Sorghum-Hirse | 30 | 75 | 85 | 95 | 100 |
| Dikotyle Unkräuter | | | | | |
| Amaranthus retroflexus | 90 | 100 | 100 | 100 | 100 |
| Chenopodium album | 85 | 95 | 100 | 100 | 100 |
| Chrysanthemum segetum | 50 | 75 | 95 | 100 | 100 |
| Galium aparine | 0 | 10 | 50 | 75 | 95 |

| | 0,25 | 0,5 | 1,0 | 2,0 | 4,0 |
|---|---|---|---|---|---|
| Matricaria inodora | 80 | 90 | 100 | 100 | 100 |
| Polygonum persicaria | 75 | 95 | 100 | 100 | 100 |
| Sinapis alba | 60 | 80 | 95 | 100 | 100 |
| Stellaria media | 70 | 90 | 95 | 100 | 100 |
| Ungräser | | | | | |
| Apera spica-venti | 40 | 55 | 60 | 75 | 75 |
| Digitaria sanguinalis | 35 | 40 | 65 | 75 | 80 |
| Poa annua | 50 | 60 | 75 | 85 | 95 |
| Setaria viridis | 45 | 50 | 70 | 80 | 80 |

Der Vergleich der Ergebnisse gemäß den Beispielen 12 bis 15 mit den Daten gemäß Vergleichsbeispiel 3 verdeutlicht die bessere Nutzpflanzenverträglichkeit der erfindungsgemäßen Wirkstoffe, insbesondere bei den Getreidearten. Darüberhinaus übertreffen die erfingunsgemäßen Herbizide deutlich die Wirksamkeit des Vergleichsmittels, vor allem gegenüber dem wichtigen Problemunkraut Galium aparine (Klettenlabkraut) sowie gegen einige Schadgräser.

Patentansprüche

1. Herbizid wirksame Zusammensetzungen, die zumindest eine Verbindung der allgemeinen Formel

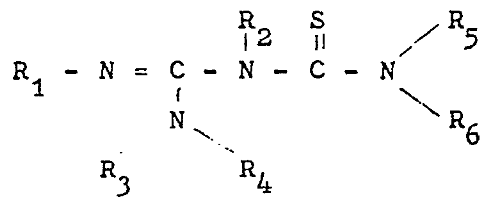

enthalten, wobei

$R_1$ und $R_2$ gleiche oder verschiedene, ggf. substituierte aromatische Reste sind, und

$R_3$, $R_4$, $R_5$ und $R_6$ für gleiche oder verschiedene Substituenten stehen, mit der Bedeutung aliphatischer Rest mit 1 bis 4 Kohlenstoffatomen, cycloaliphatischer Rest mit 5 bis 6 Kohlenstoffatomen und weiterhin, daß $R_3$ und $R_4$ sowie $R_5$ und $R_6$, ggf. ein weiteres Heteroatom enthaltende Ringe mit 4 bis 5 Kohlenstoffatomen bilden können.

2. Herbizid wirksame Zusammensetzungen nach Anspruch 1, die zumindest eine Verbindung enthalten, für die $R_1$ und $R_2$ gleiche oder verschiedene, Naphtyl- oder Phenylreste bedeuten, mit 0 bis 3 Substituenten aus der Gruppe der Halogene, der Alkylgruppen mit 1 bis 3 Kohlenstoffatomen, der substituierten Alkylgruppen mit 1 bis 3 Kohlenstoffatomen, der Mercaptogruppen mit 1 bis 3 Kohlenstoffatomen, der Carboxyalkylgruppen mit 1 bis 3 Kohlenstoffatomen, der Nitro- und der Nitrilgruppen.

3. Herbizid wirksame Zusammensetzungen nach den Ansprüchen 1 und 2, die zumindest eine Verbindung enthalten, bei der die Reste $R_3$, $R_4$, $R_5$ und $R_6$ den Methylrest bedeuten.

4. Verfahren zur Herstellung von Verbindungen der allgemeinen Formel

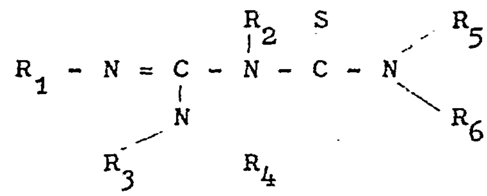

wobei

$R_1$ und $R_2$ gleiche, ggf. substituierte aromatische Reste bedeuten und

$R_3$, $R_4$, $R_5$ und $R_6$, mit der Maßgabe, daß die Kombinationen $R_3/R_4$ und $R_5/R_6$ identisch sind, für aliphatische Reste mit 1 bis 4 Kohlenstoffatomen, für cycloaliphatische Reste mit 5 bis 6 Kohlenstoffatomen stehen, sowie $R_3/R_4$ bzw. $R_5/R_6$ einen, ggf. ein weiteres Heteroatom enthaltenden Ring mit 4 bis 5 Kohlenstoffatomen bilden können,

dadurch gekennzeichnet, daß

a) Thioharnstoff der allgemeinen Formel

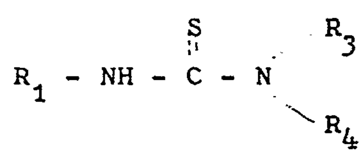

mit Chlor oder Brom in Gegenwart polarer Lösungsmittel als Flüssigphase behandelt wird und

b) das Reaktionsprodukt gemäß a) alkalisch gestellt wird.

5. Verfahren zur Herstellung von Verbindungen der allgemeinen Formel

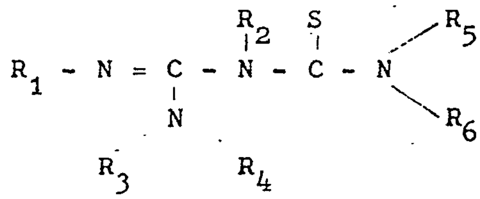

wobei

$R_1$ und $R_2$ gleiche oder verschiedene, ggf. substituierte aromatische Reste sind und

$R_3$, $R_4$, $R_5$ und $R_6$ für gleiche oder verschiedene Substituenten stehen, mit der Bedeutung aliphatischer Rest mit 1 bis 4 Kohlenstoffatomen, cycloaliphatischer Rest mit 5 bis 6 Kohlenstoffatomen und weiterhin, daß $R_3$ und $R_4$ sowie $R_5$ und $R_6$, ggf. ein weiteres Heteroatom enthaltende Ringe mit 4 bis 5 Kohlenstoffatomen bilden können,

d a d u r c h   g e k e n n z e i c h n e t ,   daß

a) Chlorformamidin der allgemeinen Formel

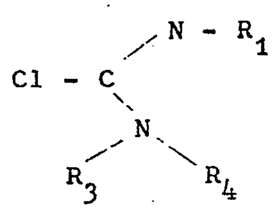

wobei

$R_1$, $R_3$ und $R_4$ die obengenannte Bedeutung haben,

mit Thioharnstoff der allgemeinen Formel

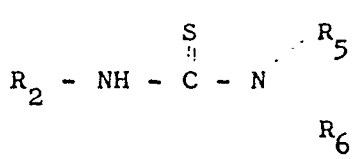

wobei

$R_2$, $R_5$ und $R_6$ die obengenannte Bedeutung haben

in Gegenwart von Chloroform umgesetzt werden und

b) das Reaktionsgemisch gemäß a) alkalisch gestellt wird.